# EUROPÄISCHE PATENTSCHRIFT

(11) **EP 0 894 608 B1**
(45) Veröffentlichungstag und Bekanntmachung des Hinweises auf die Patenterteilung: **21.03.2001**
(21) Anmeldenummer: 98112840.8
(22) Anmeldetag: 10.07.1998
(51) Int. Cl.: B29C 49/20

(54) **Verfahren zum Herstellen einer Wärmflasche und danach hergestellte Wärmflasche**
Method of manufacturing a hot water bottle and hot water bottle manufactured by this process
Procédé pour la fabrication d'une bouillotte et bouillotte obtenue par ce procédé

(30) Priorität: 18.07.1997 DE 19730800
(43) Veröffentlichungstag der Anmeldung: 03.02.1999
(73) Patentinhaber: Fashy GmbH Produktion und Vertrieb Gummi-Kraus, 70825 Korntal-Münchingen (DE)
(72) Erfinder: Frosch, Hugo, 86470 Thannhausen (DE); Giebisch, Hermann, 86381 Krumbach (DE)
(74) Vertreter: Dreiss, Fuhlendorf, Steimle & Becker

(56) Entgegenhaltungen:
- DE-A- 2 911 626
- FR-A- 2 390 268
- GB-A- 2 051 661
- US-A- 3 705 931

## Beschreibung

Die vorliegende Erfindung bezieht sich auf ein Verfahren zum Herstellen einer Flasche, insbesondere Wärmflasche, nach dem Oberbegriff des Anspruchs 1 und auf eine danach hergestellte Flasche, insbesondere Wärmflasche, nach dem Oberbegriff des Anspruchs 4.

Beispielsweise bei Wärmflaschen, die aus einem weichelastischen thermoplastischen Material geblasen werden, besteht ein Problem in der Befestigung des Verschlusseinsatzes, der aus einem harten Material sein muss, im Halsbereich. Der Verschlusseinsatz ist erstens am Ende eines Einfülltrichters vorgesehen und kann zweitens aus Gründen der Dichtheit nicht aus einem weichen, bspw. dem Flaschenmaterial sein.

Aus der DE 28 20 366 A1 ist ein Verfahren der eingangs genannten Art bekannt, bei dem der Einsatz aus einem Material bestehen soll, das oberflächig angeschmolzen werden kann, damit es dann, wenn es in Kontakt mit dem aufzublasenden Schlauch kommt, mit dem Halsbereich verschweißt wird. Dies ist ein theoretischer Ansatz, da es bisher kein Material für einen harten Einsatz gibt, das mit dem für den extrudierten Schlauch verwendeten PVC-Thermoplast in derartiger Weise eine feste Verbindung eingeht.

Andererseits ist es aus der DE 29 11 626 A1 bekannt, eine Wärmflasche aus thermoplastischem Kunststoff im Spritzgußverfahren herzustellen und dabei den separat hergestellten Verschlusseinsatz mit einzuspritzen, wobei der Verschlusseinsatz mit zwei axial im Abstand angeordneten Bohrungskränzen versehen ist, durch deren Bohrungen das Flaschenmaterial beim Spritzgußvorgang eingespritzt wird. Das Spritzgußverfahren ist jedoch im Gegensatz zum Blasverfahren sowohl verfahrensmäßig als auch im Hinblick auf das zu verwendende Werkzeug wesentlich aufwendiger.

Aufgabe der vorliegenden Erfindung ist es deshalb, ein Verfahren der eingangs genannten Art zu schaffen, mit dem während des der Herstellung der Flasche dienenden Blasverfahrens in einfacher Weise eine mechanische Verbindung zwischen dem Halsbereich der Flasche und dem Einsatz erreichbar ist. Außerdem soll eine durch das Blasverfahren hergestellte Flasche, insbesondere Wärmflasche, geschaffen werden, deren Einsatz in mechanischer Weise verankert ist.

Zur Lösung dieser Aufgabe sind bei einem Verfahren der genannten Art die im Anspruch 1 angegebenen Merkmale und bei einer Flasche der genannten Art die im Anspruch 4 angegebenen Merkmale vorgesehen.

Zweckmäßige Weiterbildungen der Erfindung sind Gegenstand der jeweiligen abhängigen Ansprüche.

Mit den erfindungsgemäßen Maßnahmen ist ohne zusätzliche Verfahrensschritte eine stabile und dichte mechanische Verankerung und homogene Verbindung des Verschlusseinsatzes im Halsbereich der Flasche erreicht. Da diese mechanische Verankerung mit dem ohnehin vorzunehmenden Verschließen der Werkzeughälften erreichbar ist, wird kein zusätzlicher Zeitaufwand benötigt.

Die bei dem mit der Flasche zu verwendenden Einsatz vorgesehen Maßnahmen gewährleisten ein optimales Eindrücken des Schlauchmaterials in und durch die Bohrungen.

Vorteilhafte Ausgestaltungen ergeben sich aus den Merkmalen eines oder mehrerer der Unteransprüche. Dabei wird insbesondere Vorsorge dafür getroffen, dass sich im Halsbereich zum Einbringen des Schlauchmaterials in die und durch die Bohrungen und in die Hinterschneidungen beim Zusammenfahren der Werkzeughälften Materialanhäufungen ergeben, die sich zur mechanischen Verankerung entsprechend verteilen können. Außerdem ist es vorteilhaft, das Schlauchmaterial gleichzeitig für eine entsprechende Dichtlippe über dem Einsatz zu verwenden.

Weitere Einzelheiten der Erfindung sind der folgenden Beschreibung zu entnehmen, in der die Erfindung anhand des in der Zeichnung dargestellten Ausführungsbeispieles näher beschrieben und erläutert ist. Es zeigen:
- Figur 1: in schematischer, teilweise längsgeschnittener und abgebrochener Darstellung den Einfüll- und Halsbereich einer aus thermoplastischem Material geblasenen Wärmflasche,
- Figur 2: den in Figur 1 in der Wärmflasche gehaltenen Einsatz in vergrößertem Längsschnitt und
- Figur 3: eine Draufsicht gemäß Pfeil III der Figur 2.

Die in der Zeichnung dargestellte und im Blasverfahren hergestellte Flasche ist eine Wärmflasche 10, die einen nur teilweise dargestellten Flaschenkorpus 11, einen Einfülltrichter 12 und einen zwischen Korpus 11 und Trichter 12 angeordneten Halsbereich 13 aufweist, in welchem ein Verschlusseinsatz 14 mechanisch verankert gehalten ist.

Während der Wärmflaschenkorpus 11 und der Einfülltrichter 12 einschließlich des Halsbereichs 13 aus einem weichelastischen, thermoplastischen Kunststoffmaterial, bspw. PVC, ist, besteht der Verschlusseinsatz 14 aus einem harten Kunststoff, wie bspw. Polyamid oder PP.

Der Verschlusseinsatz 14 ist gemäß den Figuren 2 und 3 ein ringförmiges Teil mit einem Innengewinde 16, in das in nicht dargestellter Weise ein Verschlussstopfen einschraubbar ist. Der Verschlusseinsatz 14 besitzt an seinem äußeren und inneren Endbereich einen Ringrand 17 bzw. 18, wobei die Ringränder 17 und 18 durch eine ringförmige Hinterschneidung 19 voneinander getrennt sind. Der innere Ringrand 18 ist mit einer dem gegenüberliegenden äußeren Ringrand 17 zugewandten Schrägfläche 21 versehen, die vom Außenumfang her schräg nach innen zur Innenseite des äußeren Ringrandes 17 führt. Der äußere Ringrand 17 ist mit einer Vielzahl von gleichmäßig über seinen Umfang verteilten axialen Durchgangsbohrungen 22 versehen, die von der Außenseite des äußeren Ringrandes 17 ausgehen und in die mit der Schrägfläche 21 versehene ringförmige Hinterschneidung 19 münden.

Wie aus Figur 1 zu ersehen ist, ist der Verschlusseinsatz 14 im Halsbereich 13 der Wärmflasche 10 dadurch mechanisch verankert, dass das thermoplastische Wärmflaschenmaterial in die ringförmige Hinterschneidung 19 und durch die axialen Bohrungen 22 hindurch gedrungen und mit einer an der äußeren Stirnfläche 23 des äußeren Ringrandes 17 angeordneten Dichtlippe 24 aus dem thermoplastischen Wärmflaschenmaterial einstückig ist.

Eine derartige mit einem mechanisch verankerten Verschlusseinsatz 14 versehene Wärmflasche 10 wird in der nachstehend beschriebenen Weise hergestellt. Ein aus dem betreffenden thermoplastischen Material extrudierter Schlauch wird zwischen zwei auseinandergefahrene Formhälften eines Blaswerkzeugs 26 gebracht. In den thermoplastischen Schlauch wird in Höhe dessen vorgesehenen Halsbereiches 13 der Verschlusseinsatz 14 gebracht, der am vorderen Ende eines Blasdorns 27 gehalten ist. Dabei ist der Innendurchmesser des aus weichelastischem thermoplastischem Material extrudierten Schlauches wesentlich größer als der Außendurchmesser des Verschlusseinsatzes 14. Ggf. ist auch vorgesehen, die Materialdicke des thermoplastischen Schlauches größer vorzusehen, als der endgültigen Materialdicke der Wärmflasche 10 insbesondere im Halsbereich 13 entspricht. Es werden dann die beiden Blaswerkzeugformhälften, deren Innenkontur der Außengestalt der zu blasenden Wärmflasche 10 entspricht, aufeinander zugefahren und verschlossen, wobei der extrudierte Schlauch im Halsbereich 13 zwischen die betreffenden Bereiche der Werkzeugformhälften und der Außenseite des Verschlusseinsatzes 14 gelangt. Aufgrund der Anhäufung von thermoplastischem Material beim Verschließen des Werkzeugs 26 zwischen den Werkzeughälften und dem Verschlusseinsatz 14 im Halsbereich 13 wird das thermoplastische Material in die ringförmige Hinterschneidung 19 und durch diese hindurch in und durch die axialen Bohrungen 22 eingedrückt. Dadurch werden die axialen Bohrungen 22 bei 28 ausgefüllt. Außerdem wird thermoplastisches Material in einen Bereich zwischen einem Blasdornkranz und dem äußeren Ringrand 17 des Verschlusseinsatzes 14 gebracht, so dass sich die Dichtlippe 24 ergibt. Durch das Verdrücken von heißem thermoplastischem Schlauchmaterial in diese Bereiche, ergibt sich eine einstückige, d.h. homogene Verbindung des Wärmflaschenhalsbereiches, der Dichtlippe 24 und des in der ringförmigen Hinterschneidung 19 und den axialen Bohrungen 22 befindlichen Schlauchmaterials, so dass der Verschlusseinsatz 14 im Halsbereich 13 der Wärmflasche 10 mechanisch verankert ist. Danach oder parallel hierzu wird über den Blasdorn 27 Luft eingeblasen, so dass sich das Schlauchmaterial ausdehnt und zum Korpus 11 im Werkzeug dadurch geformt wird, dass sich der Schlauch an die Innenkontur des Blasformwerkzeugs 26 anlegt. Der Trichter 12 wird beim Verschließen der Form gepresst (kalibriert). Nach dem Erstarren des thermosplastischen Materials werden die Formhälften geöffnet und die fertig geformte, weichelastische Wärmflasche 10 mit dem mechanisch verankerten Verschlusseinsatz kann entnommen werden.

Gemäß einem nicht dargestellten Ausführungsbeispiel sind die beiden Wärmflaschenhälften des Wärmflaschenkorpus 11 in zwei verschiedenen Farben oder in opaker und durchsichtiger Form o.dgl. geblasen.

## Patentansprüche

1. Verfahren zum Herstellen einer mit einem vorzugsweise Innengewindeeinsatz (14) aus einem harten vorzugsweise Kunststoffmaterial versehenen Flasche (10), insbesondere Wärmflasche, aus einem aus weichelastischem, thermoplastischem Material extrudierten Schlauch im Blasverfahren, bei dem der aufzublasende Schlauch zwischen zwei geöffnete Werkzeugformhälften (26) und in den Schlauch der Einsatz (14) mit Hilfe eines Blasdorns (27) gebracht wird, **dadurch gekennzeichnet**, dass beim Zusammenfahren der beiden Werkzeughälften (26) der Schlauch zwischen einen Halsbereich (13) der Werkzeughälften (26) und dem Einsatz (14) derart gequetscht wird, dass das heisse Schlauchmaterial in von einer ringförmigen Hinterschneidung (19) des Einsatzes (14) ausgehende axiale Bohrungen (22) eines Ringrandes (17) gedrückt wird.

2. Verfahren nach Anspruch 1, dadurch gekennzeichnet, dass der extrudierte Schlauch einen erheblich größeren Durchmesser aufweist als der Einsatz (14).

3. Verfahren nach Anspruch 1 oder 2, dadurch gekennzeichnet, dass der extrudierte Schlauch zumindest im zu formenden Halsbereich (13) dicker ist als die Materialdicke des den Einsatz (14) umgebenden fertigen Flaschenbereichs.

4. Nach dem Verfahren nach einem der Ansprüche 1 bis 3 im Blasverfahren hergestellte Flasche, insbesondere Wärmflasche (10), mit einem im Einfüllbereich (12) gehaltenen vorzugsweise Innengewindeeinsatz (14), dadurch gekennzeichnet, dass der Einsatz (14) an einem Ringrand (17) mit über dessen Umfang verteilten, axialen Bohrungen (22) versehen ist, die in eine Hinterschneidung (19) münden, und dass die Hinterschneidung (19) mit einer Einlaufschräge (21) versehen ist.

5. Flasche nach Anspruch 4, dadurch gekennzeichnet, dass der Ringrand (17) mit den axialen Bohrungen (22) dem Flaschentrichter (12) zugewandt ist und dass die in die Hinterschneidung (19) mündende Einlaufschräge (21) von der hierzu abgewandten Seite ausgeht.

6. Flasche nach Anspruch 5, dadurch gekennzeichnet, dass die der Einlaufschräge (21) abgewandte Ringfläche des Ringrandes (17) von einer aus dem Schlauchmaterial geformten Dichtlippe (24) abgedeckt ist.

7. Flasche nach mindestens einem der Ansprüche 4 bis 6, dadurch gekennzeichnet, dass die beiden Wärmflaschenhälften des Wärmflaschenkorpus (11) aus zwei verschiedenen Farben geblasen sind.

## Claims

1. A process for producing a bottle (10), in particular a hot-water bottle, which is provided with a preferably internally screwthreaded insert (14) comprising a hard preferably plastics material, from a tube extruded from soft-elastic, thermoplastic material, by a blowing process, wherein the tube to be blown up is brought into position between two opened mould tool halves (26) and the insert (14) is brought into the tube by means of a blowing mandrel (27) characterised in that when the two tool halves (26) are moved together the tube is squeezed between a neck region (13) of the tool halves (26), and the insert (14) in such a way that the hot tube material is urged into axial bores (22) in an annular edge portion (17), the bores extending from an annular undercut portion (19) of the insert (14).

2. A process according to claim 1 characterised in that the extruded tube is of considerably larger diameter than the insert (14).

3. A process according to claim 1 or claim 2 characterised in that the extruded tube is thicker at least in the neck region (13) to be shaped than the material thickness of the finished region of the bottle around the insert (14).

4. A bottle produced by the process according to one of claims 1 to 3 in a blowing procedure, in particular a hot-water bottle (10), having a preferably internally screwthreaded insert (14) held in the filling region (12), characterised in that the insert (14) is provided at an annular edge portion (17) with axial bores (22) which are distributed over the periphery thereof and which open into an undercut portion (19) and that the undercut portion (19) is provided with an entry slope (21).

5. A bottle according to claim 4 characterised in that the annular edge portion (17) with the axial bores (22) is towards the flared mouth portion (12) of the bottle and that the entry slope (21) which communicates with the undercut portion (19) starts from the side which is remote in relation thereto.

6. A bottle according to claim 5 characterised in that the annular surface of the annular edge portion (17), which is remote from the entry slope (21), is covered by a sealing lip (24) formed from the tube material.

7. A bottle according to at least one of claims 4 to 6 characterised in that the two halves of the body (11) of the hot-water bottle are blown from two different colours.

## Revendications

1. Procédé pour la fabrication d'une bouteille (10), comportant une douille taraudée intérieure (14), en matière synthétique de préférence dure, en particulier d'une bouillotte, constituée d'un tuyau en matière thermoplastique souple et élastique réalisé par un procédé de soufflage, dans lequel le tuyau devant être soufflé est placé entre deux moitiés de moule (26) ouvertes et dans lequel on met en place la douille (14) à l'aide d'un mandrin de soufflage (27), caractérisé en ce que, lors du mouvement de rapprochement des deux moitiés de moule (26), le tuyau est serré, entre une collerette (12) des deux moitiés de moule (26) et la douille (14), de telle manière que la matière chaude du tuyau est comprimée dans des perçages axiaux (22) aménagés dans un bord annulaire (17) et partant d'une contre-dépouille (19) annulaire de la douille (14).

2. Procédé selon la revendication 1, caractérisé en ce que le tuyau extrudé a un diamètre beaucoup plus grand que celui de la douille (14)

3. Procédé selon la revendication 1 ou 2, caractérisé en ce que le tuyau extrudé est, au moins dans la collerette (13) à mouler, plus épais que l'épaisseur de la matière de la zone de la bouteille terminée qui entoure la douille (14).

4. Bouteille, en particulier bouillotte (10), fabriquée par soufflage conformément au procédé selon l'une quelconque des revendications 1 à 3 et comportant une douille taraudée intérieure (14) maintenue , de préférence, dans la zone de remplissage (12) , caractérisée en ce que la douille (14) est pourvue, sur un bord annulaire (17), de perçages axiaux (22) qui sont répartis sur la périphérie de ce dernier et débouchent dans une contre-dépouille (19), et en ce que la contre-dépouille (19) est pourvue d'un chanfrein d'entrée (21).

5. Bouteille selon la revendication 4, caractérisée en ce que le bord annulaire (17) comportant les perçages axiaux (22) est dirigé vers l'entonnoir de la bouteille (12), et en ce que le chanfrein d'entrée (21) débouchant dans la contre-dépouille (19) part du côté qui lui est opposé.

6. Bouteille selon la revendication 5, caractérisée en ce que la surface annulaire du bord annulaire (17) du côté opposé au chanfrein d'entrée (21) est recouverte d'une lèvre d'étanchéité moulée avec la matière du tuyau.

7. Bouteille selon l'une quelconque des revendications 4 à 6, caractérisée en ce que les deux moitiés de la bouillotte du corps (11) de la bouillotte sont soufflées à partir de deux couleurs différentes.
